# FASCICULE DE BREVET EUROPEEN

(11) **EP 0 427 588 B1**
(45) Date de publication et mention de la délivrance du brevet: **12.07.1995**
(21) Numéro de dépôt: 90403014.5
(22) Date de dépôt: 25.10.1990
(51) Int. Cl.: A61M 5/148, A61J 1/00

(54) **Conditionnement pour l'administration de liquides stériles, notamment liquides pharmaceutiques, nutritifs ou énergétiques**
Anlage zur Verabreichung steriler Flüssigkeiten, insbesondere pharmazeutischer, nahrhafter oder energetischer Flüssigkeiten
System for the administration of sterile liquids, especially pharmaceutical, nutritive or energetic

(30) Priorité: 26.10.1989 FR 8914037
(43) Date de publication de la demande: 15.05.1991
(73) Titulaire: Faure, Jean-Marie, F-75016 Paris (FR)
(72) Inventeur: Faure, Jean-Marie, F-75016 Paris (FR)
(74) Mandataire: CABINET BONNET-THIRION

(56) Documents cités:
- EP-A- 0 077 189
- WO-A-89/00868
- DE-A- 3 446 909
- DE-C- 3 507 818
- GB-A- 2 197 691
- US-A- 3 832 067
- US-A- 4 193 398
- US-A- 4 913 702

## Description

La présente invention concerne un conditionnement pour l'administration de liquides stériles, notamment pharmaceutiques, nutritifs ou énergétiques.

Elle est dirigée plus particulièrement, vers un conditionnement fonctionnant sous l'action d'un piston fluide.

Un exemple de conditionnement de ce type est décrit dans le document GB 2 197 691. Il est constitué par un boîtier rigide qui comporte un orifice d'entrée et un orifice de sortie et dans lequel il est prévu de loger deux poches en matériau élastique déformable, chaque poche comportant une ouverture apte à être reliée à l'un des orifices d'entrée ou de sortie du boîtier.

En fonctionnement, la première poche contient par exemple le liquide à administrer au patient et son ouverture est reliée à l'orifice de sortie du boîtier auquel on adapte un moyen quelconque pour amener le liquide au patient. L'autre poche est reliée à l'orifice d'entrée du boîtier par lequel est introduit, avec un débit choisi, un liquide dit de commande.

Ainsi, en fonctionnement, la paroi de la poche contenant le liquide de commande sollicite la paroi de la poche contenant le liquide à administrer et provoque l'expulsion de ce dernier par l'orifice de sortie avec un débit proportionnel.

Ce type de conditionnement à piston fluide permet de distribuer un liquide avec une bonne précision. Il est plus simple et moins coûteux à fabriquer que les conditionnements à pistons mécaniques.

Toutefois, par rapport à ces derniers, il convient de prendre des précautions supplémentaires. En effet, il faut éviter tout risque de contamination du liquide à administrer par le fluide de commande, car on comprend bien qu'administrer un produit souillé au patient peut avoir des conséquences graves.

Une solution pour tenter d'éviter ceci consiste, comme dans le dispositif décrit plus haut, à prévoir deux poches distinctes étanches avec chacune une paroi.

Chaque paroi constitue une barrière de sécurité. En cas de fuites dans l'une quelconque des parois des poches, le liquide qui fuit se répand dans le boîtier.

Cette solution n'est toutefois pas entièrement satisfaisante car, d'une part le boîtier, qui n'est pas jetable, doit être nettoyé pour un autre usage, et, d'autre part, surtout, rien n'est prévu pour prévenir le personnel soignant de la détérioration du conditionnement. La situation risque en outre de s'aggraver et les risques de fuites dans l'autre poche d'augmenter. Ainsi, les efforts pour empêcher la contamination auront été vains.

On connaît également des conditionnements à piston fluide qui fonctionnent sous l'action d'un piston gazeux, comme par exemple ceux décrits dans le document EP 77189. Les mêmes problèmes de contamination existent. Cependant, les solutions proposées en cas de fuites ne sont pas différentes de celles prévues pour un piston liquide : on prévoit deux chambres ou poches distinctes, de sorte que si l'une des parois n'est plus étanche, l'autre paroi joue le rôle de seconde barrière.

Jusqu'à présent, on s'est contenté de prévoir une solution au problème de contamination mais en revanche le problème de l'avertissement du personnel soignant n'a pas été soulevé.

Il s'agit là pourtant d'un aspect primordial pour que ces conditionnements à piston fluide soient effectivement fiables et sans danger pour le patient.

La présente invention se propose d'apporter une solution simple et peu coûteuse, tout en conservant les avantages des conditionnements déjà existants à piston fluide.

Elle propose un conditionnement à piston fluide pour une lente administration à un patient de volumes extrêmement précis d'une substance liquide stérile comprenant une première chambre qui est prévue pour recevoir un fluide de commande et qui comporte à cet effet une ouverture apte à être reliée à un régulateur de débit, ladite première chambre comportant une paroi souple mobile apte à solliciter une paroi souple mobile d'une seconde chambre distincte de la première chambre qui est prévue pour contenir ladite substance liquide à administrer et qui comporte à cet effet une ouverture, caractérisé en ce qu'il est prévu une troisième chambre, confinée entre les parois souples et mobiles, définissant un réservoir pour recueillir le fluide de commande et/ou la substance liquide à administrer en cas de fuites dans l'une quelconque desdites parois, et contenant un agent de détection qui fournit une indication visible en cas de fuite.

Les avantages et caractéristiques de l'invention ressortiront de la description qui va suivre faite en regard des dessins annexés sur lesquels :
la figure 1 représente en élévation une forme de réalisation d'un conditionnement selon l'invention;
la figure 2 est une vue en coupe selon la ligne II-II de la figure 1 ;
la figure 3 est une vue en coupe selon la ligne III-III de la figure 2 ;
la figure 4 représente schématiquement une autre forme de réalisation possible ;
la figure 5 est une vue en perspective du conditionnement de la figure 1 logé dans un boitier adapté pour le transfert et muni d'un couvercle de fermeture.

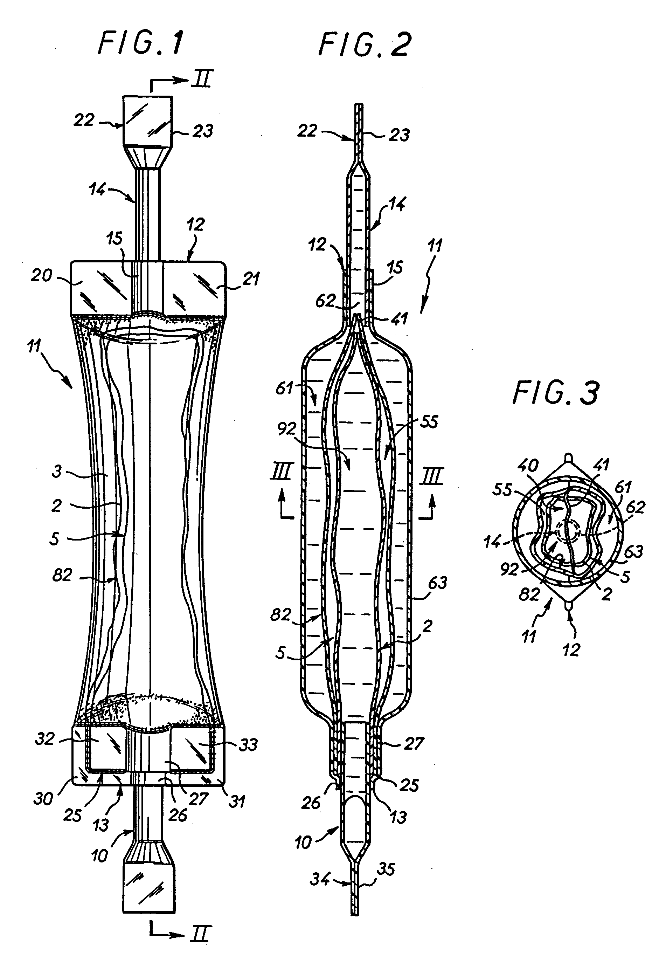

Dans la forme de réalisation illustrée aux figures 1 à 3, le conditionnement comprend une chambre 61 avec une paroi continue 63 munie d'un orifice 62 de communication avec l'extérieur (voir figure 2). La paroi 63 de la chambre 61 définit une cavité intérieure de volume constant. Elle renferme une poche étanche 2 à double paroi 5 et 82, chacune continue, souple et mobile, la paroi 5 délimitant à son tour une cavité interne ou chambre 92 de volume variable qui communique avec l'extérieur par un conduit 10 débouchant à l'extérieur de la chambre 61.

Cette chambre 61 est formée par exemple à partir d'un tube 11 souple et transparent en un matériau thermoplastique.

La première zone d'extrémité 12 du tube 11 résulte d'un thermosoudage par rapprochement et soudure selon un plan de la surface intérieure de la paroi 63. Ce rapprochement respecte le passage d'un tronçon de tuyau 14 qui vient se raccorder de manière étanche à l'orifice 62.

Ainsi la zone d'extrémité 12 comporte une zone centrale 15 globalement cylindrique résultant de la soudure étanche du tube 11 sur le pourtour d'une partie du tronçon du tuyau 14. Cette zone centrale 15 se continue de chaque côté par une aile globalement aplatie 20 et 21 (voir figure 1).

Le tuyau 14, à son extrémité opposée à celle raccordée à l'orifice 62, comporte un moyen amovible d'obturation hermétique 22. Ce moyen est réalisé par obturation du tuyau 14 par écrasement et thermosoudage du matériau sur lui-même donnant une soudure 23. Il est amovible du fait qu'il peut être facilement éliminé par coupure du tuyau 14 au-dessous de la soudure 23.

Bien entendu tout moyen amovible d'obturation hermétique d'un tuyau souple peut être utilisé notamment bouchons ou éléments de serrage. Le tuyau 14 comme le conduit 10 peut être en totalité ou en partie en un élastomère, facilement perçable par exemple et d'une manière étanche, par une aiguille simple ou double.

La seconde zone d'extrémité 13 du tube 11 résulte d'un thermosoudage du tube 11 sur une première zone d'extrémité 25 de la poche étanche 2 se présentant initialement, avant toute soudure, sous la forme d'un double manchon cylindrique à parois 5 et 82 minces.

Les zones d'extrémité 13 et 25 obtenues par rapprochement et soudure des faces en vis-à-vis du tube 11 et de la poche 2 respectent le passage d'un tronçon du conduit 10 qui traverse ainsi à un endroit déterminé la paroi 63 de la chambre 61 sans rompre l'étanchéité de cette dernière à cet endroit.

Ainsi les zones d'extrémité 13 et 25 comportent respectivement une zone centrale 26 et 27 résultant de la soudure étanche du tube 11 sur la poche étanche 2 cette dernière étant elle-même soudée de manière étanche sur le pourtour d'une partie d'un tronçon du conduit 10.

Les zones centrales 26 et 27 globalement cylindriques se continuent de chaque côté par des ailes aplaties 30, 31, 32, 33 respectivement du tube 11 et du manchon de la poche 4 (voir figure 1).

De préférence, le tube 11, le tuyau 14, et le conduit 10 sont disposés sensiblement de manière coaxiale.

Le tronçon de conduite 10 comporte, à son extrémité extérieure au tube 11, un moyen amovible d'obturation hermétique 34 ayant les mêmes caractéristiques que le moyen d'obturation 22 exposé ci-dessus, et formant une soudure 35.

La poche 2 comporte une seconde zone d'extrémité 40 thermosoudée par une thermosoudure 41, simultanément, sur les deux parois 5 et 82.

Cette dernière soudure isole de manière étanche la chambre 92 de la poche 2 et crée une troisième cavité étanche ou chambre 55 entre les deux parois 5 et 82.

En fonctionnement, les connexions se font directement, par le conduit 14 à un régulateur de débit lorsque le conditionnement est livré, chambres 61 et 92 pleines, respectivement, du liquide de commande et du liquide stérile à administrer.

On peut donner des attributions inverses aux deux chambres 61 et 92 stériles, c'est-à-dire que l'on peut avoir le liquide de commande en 92 et liquide stérile à administrer en 61. Ceci ajoute un intérêt supplémentaire à savoir, celui de pouvoir choisir un matériau différent pour la première chambre 61 de celui constituant la poche 2. On peut résoudre ainsi les éventuels problèmes d'incompatibilité entre le matériau de la poche 2 qui doit être souple ou élastique et le produit contenu dans cette poche. On peut donc choisir pour la chambre 61 tout matériau approprié, par exemple verre, métal céramique, matière plastique.

Dans ce mode de réalisation, la poche 2 est représentée et décrite comme ayant une double paroi. Elle peut selon l'invention avoir une paroi multiple.

Le conditionnement obtenu comprend donc une chambre 61 et à l'intérieur de celle-ci une poche déformable 2, la chambre et la poche n'ayant aucune communication entre elles mais communiquant, individuellement et séparément, avec l'extérieur par au moins une ouverture, respectivement 62 et 10, de telle sorte que toute variation du volume de l'une entraîne la variation d'un volume égal de l'autre.

Dans l'exemple de réalisation choisi, les deux chambres 61 et 92 ne communiquent avec l'extérieur que par une seule ouverture, respectivement tuyau 14 et conduit 10. Cet exemple n'est pas limitatif et le nombre d'ouvertures peut être quelconque et multiple.

D'autre part, ces deux ouvertures sont fermées, la poche 2 et la chambre 61 contenant respectivement et chacune un liquide. Si la poche 2 et la chambre 61 sont commercialisées vides le conditionnement peut être commercialisé avec une seule fermeture, fermeture du volume qui doit être stérile, volume devant servir à l'administration du liquide stérile. Il en est de même si le conditionnement est commercialisé, contenant dans la poche 2 ou dans la chambre 61, un seul liquide à savoir le liquide à administrer, ou un médicament ou un aliment, ou un produit énergétique sous forme solide.

Le volume et le dosage en médicament ou autre du liquide remplissant la chambre 61 ou 92 sont déterminés avec précision et peuvent être inscrits par tout moyen sur le tube 11. Si la poche 2 et la chambre 61 sont commercialisées vides il est également possible de ne faire aucune fermeture, le conditionnement étant alors livré dans un sachet soudé, pelable, l'ensemble étant stérilisé.

Toutes les solutions, plus ou moins combinées sont possibles si le tube 11 est de diamètre raisonnable : ainsi, la soudure 12 peut être supprimée, le tuyau 14 également et la connexion de la source du fluide de commande se fait directement sur ce tube 11.

Dans cet exemple, également, les thermosoudures de la poche 2 et de la chambre 61 sont faites simultanément autour et sur le conduit 10.

On peut par exemple souder d'abord la paroi 5, puis la paroi 82 de la poche 2, puis la paroi 63 de la chambre 61, soit l'une au-dessus l'une de l'autre, soit à des positions successives et différentes sur la longueur du conduit 10.

La poche 2 et le conduit 10 peuvent être en une seule pièce et il ne reste plus alors qu'une seule thermosoudure à faire, celle de la chambre 61 sur la partie convenable de la poche 2.

Dans cet exemple également, la thermosoudure se fait sur des tubes cylindriques. Le conditionnement selon l'invention peut être fabriqué par tout procédé connu tel que moulage, soufflage, collage, ou autres. La poche elle, peut être également fabriquée selon les procédés utilisés pour préservatifs, les gants en plastique ou latex, les poches de solutés grand volume.

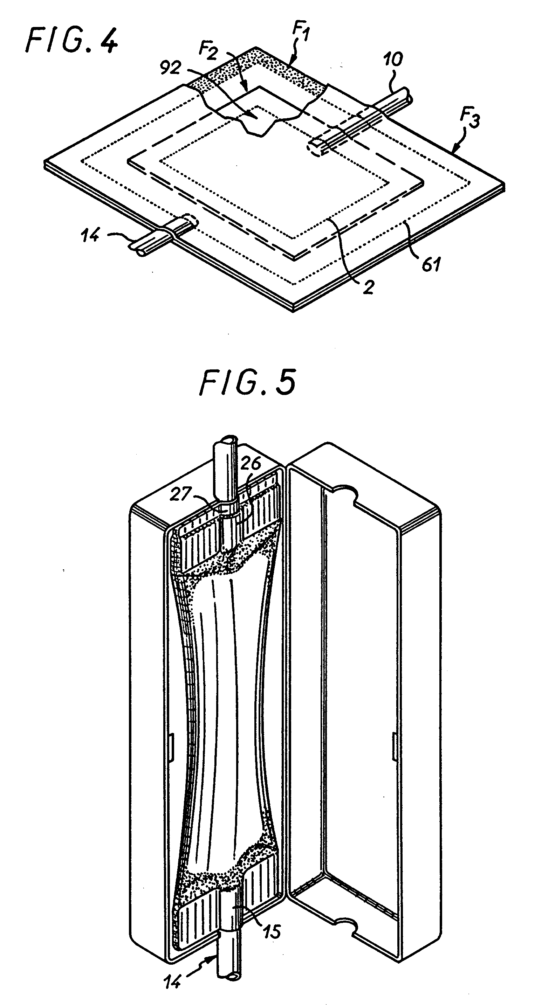

A titre de nouvel exemple utilisant la thermosoudure, on peut par exemple ne plus partir de tubes mais de simples feuilles. Cet exemple est illustré sur la figure 4. On part de deux feuilles F₁, F₂ de dimensions différentes et, dans un premier temps, on thermosoude, tout autour de son périmètre de même que sur et autour du conduit 10 la plus petite des feuilles sur la plus grande. On obtient ainsi la poche 2 avec sa chambre 92. dans un deuxième temps, on thermosoude, tout autour de son périmètre et au-dessus du tuyau 14, ainsi qu'au-dessus et autour du conduit 10, une troisième feuille F₃ de mêmes dimensions que la plus grande et on obtient la chambre 61.

Pour que la poche 2 ait une double paroi on utilise soit deux petites feuilles de même taille déjà thermosoudées entre elles, soit thermosoudées simultanément, ou bien l'une après l'autre, autour et sur le conduit 10 sur la grande feuille.

Lorsque l'administration arrive à son terme, il est possible de remplir de nouveau le conditionnement d'un second liquide stérile à administrer. Pour réaliser cette opération le conduit 10 est fermé de l'extérieur par un élément de serrage. Le conduit 10 étant dans ce cas en un élastomère relativement épais ; il est alors piqué par une aiguille creuse reliée à un réservoir contenant le second liquide, de façon que cette aiguille débouche dans la lumière du conduit 10 en communication de fluide avec la chambre 92. On remplit alors la chambre 92 soit en exerçant une légère surpression sur le liquide stérile avec évacuation concomitante de la chambre 61 du liquide de commande, soit par aspiration du liquide de commande entraînant l'aspiration dans la chambre 92 du liquide stérile. Pendant ce remplissage le tuyau 10 étant fermé aucun liquide n'est administré au patient. A la place de la connexion par piqûre du conduit 10, tout autre moyen de connexion temporaire, notamment robinets à trois voies, bouchons perforables, embouts Luer en T, peut être utilisé.

Lorsque le remplissage de 92 est terminé, le raccordement avec ledit réservoir contenant le second liquide, est supprimé ou obturé et l'administration est reprise chez le patient sous l'action du fluide de commande.

Parmi diverses possibilités de réalisation on peut notamment envisager les dispositions suivantes : un moyen de fermeture réversible dudit orifice ou dudit conduit afin de fermer hermétiquement l'une desdites chambres 61, 92.

Le régulateur de débit, qu'il soit connecté à l'une ou l'autre des cavités, peut être un tube capillaire calibré ayant un diamètre, une longueur et un angle déterminés ou, tout simplement, un micro-orifice de diamètre calibré. Ce micro-orifice peut être fait dans la paroi même de la chambre et/ou de la poche ; il constitue alors, leur, ou une de leurs ouvertures avec l'extérieur. Ce micro-orifice, quel que soit le matériau utilisé est avantageusement réalisé avec une très grande précision au laser.

Dans tous les cas d'utilisation d'un piston fluide il peut y avoir un risque de fuite dans la paroi de la poche, et donc un danger certain pour le patient qu'une partie de ce piston fluide lui soit ainsi administré, et ce, sans que ce fait puisse être perçu.

Pour éliminer ce danger, le conditionnement selon l'invention met en oeuvre, un moyen pour détecter une fuite dans l'une quelconque des parois mobiles pour prévenir le personnel soignant.

Ce moyen est un moyen indicateur transformant le fait non perceptible constitué par une fuite en phénomène perceptible.

Il peut être solide, liquide, gazeux, soluble dans le liquide de commande et/ou dans le liquide à administrer ; insoluble dans le fluide de commande et/ou dans le liquide à administrer, constitué d'un seul élément ou de plusieurs éléments.

Le phénomène produit est perceptible visuellement par la disparition et/ou le changement d'une ou plusieurs couleurs et/ou la modification de transparence ou limpidité, ou de tout autre paramètre physique décelable soit automatiquement en mettant en oeuvre un appareillage plus ou moins sophistiqué, émettant à titre d'exemple un signal de toute nature perceptible.

L'agent de détection peut être un colorant placé dans la cavité 55, en solution ou sous forme solide, ou une simple inscription sur l'une des parois (on sait ce qui se passe quand une goutte d'eau ou une goutte de pluie tombe sur une feuille où l'on a écrit avec un stylo) ou en poudre fine, en comprimé, fixé sur la paroi, fixé sur un autre support collé à la paroi ou libre dans la chambre 55.

S'il y a par exemple une fuite dans la paroi 82 on observera une coloration en 61 et 55 et s'il y a une fuite dans la paroi 5 on observera une coloration en 92 et 55.

On remarquera que l'on sait ainsi quelle paroi est défectueuse et de plus, cette indication visuelle a un caractère préventif, car au moment ou une des parois de la chambre 55 a une fuite, il n'y a, à ce moment là, aucune communication entre 61 et 92.

Si ce colorant est peint, inscrit ou dessiné sur la paroi on aura la disparition de cette inscription.

Le détecteur peut être un produit qui réagit avec le fluide de commande ou et avec le liquide à distribuer. Ce peut être selon une réaction chimique, enzymatique, ou autre.

Par exemple l'agent indicateur de fuites est fixé sur un support cellulosique dans la chambre 55 ou sur une de ses parois. Ce produit étant ainsi à l'état solide et insoluble dans les deux liquides, liquide de commande et liquide à distribuer, s'il y a une fuite dans une des parois 5, 82 il n'y aura aucun passage de ce produit en 61 et 92. Ce produit peut être, par exemple, l'orthotoluidine-glucose-oxydase-péroxydase du raifort. Les deux liquides sont glucosés. S'il y a une fuite de 82 ou 5 il y a immédiatement coloration du produit.

L'indication d'une fuite peut être produite par un changement de couleur : si le produit fixé est cette fois, par exemple du chlorure de cobalt bleu à l'état sec et s'il y a une fuite de la paroi 82 ou 5 de l'eau arrive en contact avec ce produit et sa teinte vire au lilas puis au rose.

L'indication d'une fuite peut être aussi signalée par la disparition d'une couleur. Si sur le même support on met deux produits réagissant entre eux, s'ils sont secs il ne se produira rien, mais s'il y a une fuite de 82 ou 5 l'eau pénètre dans la chambre 55, imbibe le support, met les deux produits en contact et la réaction se produit.

Par exemple si on a mis un seul calcique coloré par l'O-Crésol-phtaléine et comme 2ème produit du "Complexon", il y aura décoloration.

L'agent indicateur de fuite peut être gazeux, par exemple de l'anhydrique carbonique. Si le piston fluide est un liquide il peut être dissous dans ce liquide. Si en 55 on a de l'eau de chaux, en cas de fuite en 82 il se produira un trouble de ce liquide. si l'on a mis un produit réagissant avec CO2 on aura une coloration, par exemple produit à base de phosphoenol pyruvate. Cela peut être aussi des vapeurs d'alcool selon le principe bien connu par les automobilistes soumis au "test du ballon".

Dans ces exemples, il est question de couleurs, mais cela peut être tout rayonnement, toute modification électrique et tous les exemples décrits peuvent être combinés à volonté.

Il est à noter que cet agent indicateur de fuites a un autre intérêt particulier non négligeable , à savoir, si un produit y est préconditionné, celui de signaler, avant l'utilisation du conditionnement, une fuite dans une des parois et un rejet de ce conditionnement car il y aurait un risque de mauvaise conservation du produit conditionné et en tout cas un risque pour le patient.

A noter également que la double paroi, selon l'invention augmente considérablement la durée de conservation.

D'un point de vue pratique pour un conditionnement selon l'invention, positionné transportable, il peut être prévu un boîtier protecteur indéformable 60 comme représenté à titre d'exemple à la figure 5.

En effet, le conditionnement comporte un tube 11 pouvant se déformer par un écrasement accidentel sous l'effet d'une force s'appliquant sur le tube pour en diminuer le volume. Cette diminution de volume pourrait entraîner une brutale accélération de l'injection et doit être absolument évitée. Le logement du conditionnement dans le boîtier 60 refermé permet donc d'éviter toute pression accidentelle sur le tube 11.

Bien entendu dans le cas où la chambre 61 est réalisée avec une paroi 63 suffisamment épaisse et indéformable cette protection par boîtier peut apparaître superflue.

## Revendications

1. Conditionnement à piston fluide pour une lente administration à un patient de volumes extrêmement précis d'une substance liquide stérile comprenant une première chambre (61,92) qui est prévue pour recevoir un fluide de commande et qui comporte à cet effet une ouverture (10,14) apte à être reliée à un régulateur de débit (1), ladite première chambre comportant une paroi souple mobile (5,82) apte à solliciter une paroi souple mobile (5,82) d'une seconde chambre (61,92) distincte de la première chambre (61,92) qui est prévue pour contenir ladite substance liquide à administrer et qui comporte à cet effet une ouverture (10,14), caractérisé en ce qu'il est prévu une troisième chambre (55), confinée entre les parois souples et mobiles (5,82), définissant un réservoir pour recueillir le fluide de commande et/ou la substance liquide à administrer en cas de fuites dans l'une quelconque desdites parois, et contenant un agent de détection qui fournit une indication visible en cas de fuite.

2. Conditionnement selon la revendication 1, caractérisé en ce que ledit agent de détection est fixé sur l'une des parois (5,82) de ladite troisième chambre.

3. Conditionnement selon la revendication 1, caractérisé en ce que ledit agent de détection se trouve en liberté dans ladite troisième chambre (55).

4. Conditionnement selon l'une quelconque des revendications 1 à 3, caractérisé en ce que ledit agent de détection est apte à modifier la couleur du fluide qui pénètre dans ladite troisième chambre en cas de fuite.

5. Conditionnement selon l'une quelconque des revendications 1 à 3, caractérisé en ce que ledit agent de détection est apte à troubler le liquide qui pénètre dans ladite troisième chambre en cas de fuite.

6. Conditionnement selon la revendication 5, caractérisé en ce que ledit agent est à l'état gazeux.

7. Conditionnement selon l'une quelconque des revendication 1 à 6, caractérisé en ce qu'il constitué par une première poche (61) à paroi souple (63) contenant une seconde poche étanche (2) à paroi double (5,82), ladite première poche (61) définissant soit la première chambre soit la deuxième chambre tandis que la seconde poche (2) définit l'autre chambre, sa paroi double (5,82) définissant ladite troisième chambre (55).

8. Conditionnement selon la revendication 7, caractérisé en ce que les parois de ladite seconde poche sont des parois multiples.

9. Conditionnement selon l'une quelconque des revendications 1 à 8, caractérisé en ce que lesdites ouvertures (10,14) sont disposées à l'opposé l'une de l'autre.

10. Conditionnement selon la revendication 10, caractérisé en ce que, à l'une des extrémités du conditionnement, un tronçon de tuyau (14) est raccordé de manière étanche à la paroi (63) de ladite première poche (61) et permet l'accès à la chambre définie par celle-ci, tandis qu'à l'autre extrémité dudit conditionnement, un conduit (10) permet l'accès à l'autre chambre (92) définie par la seconde poche (2), la paroi double (5,82) de cette dernière ainsi que la paroi (63) de la première poche étant raccordées de manière étanche à ce conduit (10).

11. Conditionnement selon l'une quelconque des revendications précédentes, caractérisé en ce qu'il est prévu plusieurs ouvertures de communication avec l'extérieur dans lesdites première et seconde chambres.

## Claims

1. A pack of fluid piston type for slow administration to a patient of extremely precise volumes of a sterile liquid substance, comprising a first chamber (61, 92) which is intended to receive a control fluid and which for that purpose comprises an opening (10, 14) for being connected to a flow regulator (1), said first chamber comprising a movable flexible wall (5, 82) capable of acting on a movable flexible wall (5, 82) of a second chamber (61, 92) separate from the first chamber (61, 92), which is provided for containing said liquid substance to be administered and which for that purpose comprises an opening (10, 14), characterised in that there is provided a third chamber (55) which is confined between the movable flexible walls (5, 82), defining a reservoir for collecting the control fluid and/or the liquid substance to be administered in the event of leaks in either one of said walls, and containing a detection agent which provides a visible indication in the event of a leak.

2. A pack according to claim 1 characterised in that said detection agent is fixed on one of the walls (5, 82) of said third chamber.

3. A pack according to claim 1 characterised in that said detection agent is disposed free in said third chamber (55).

4. A pack according to any one of claims 1 to 3 characterised in that said detection agent is capable of modifying the colour of the fluid which passes into said third chamber in the event of a leak.

5. A pack according to any one of claims 1 to 3 characterised in that said detection agent is capable of clouding the liquid which passes into said third chamber in the event of a leak.

6. A pack according to claim 5 characterised in that said agent is in the gaseous state.

7. A pack according to any one of claims 1 to 6 characterised in that it is formed by a first bag (61) having a flexible wall (63) containing a second sealed bag (2) with a double wall (5, 82), said first bag (61) defining either the first chamber or the second chamber while the second bag (2) defines the other chamber, its double wall (5, 82) defining said third chamber (55).

8. A pack according to claim 7 characterised in that the walls of said second bag are multiple walls.

9. A pack according to any one of claims 1 to 8 characterised in that said openings (10, 14) are disposed opposite to each other.

10. A pack according to claim 9 characterised in that, at one of the ends of the pack, a pipe portion (14) is sealingly connected to the wall (63) of said first bag (61) and permits access to the chamber defined by sane while at the other end of the pack a conduit (10) permits access to the other chamber (92) defined by the second bag (2), the double wall (5, 82) of the latter and the wall (63) of the first bag being sealingly connected to said conduit (10).

11. A pack according to any one of the preceding claims characterised in that there are provided a plurality of openings for communicating with the exterior in said first and second chambers.

## Patentansprüche

1. Anlage mit Flüssigkeitskolben zur langsamen Verabreichung einer sterilen flüssigen Substanz mit äußerst genauen Volumenbeträgen an einen Patienten, mit folgenden Merkmalen:
eine erste Kammer (61, 92) ist zur Aufnahme einer Steuerflüssigkeit vorgesehen und enthält zu diesem Zweck eine Öffnung (10, 14), die zur Verbindung mit einem Durchsatzregler (1) verbindbar ist; die erste Kammer enthält eine bewegliche biegsame Trennwand (5, 82), die in der Lage ist, eine bewegbare biegsame Trennwand (5, 82) einer zweiten Kammer (61, 92) zu belasten, die sich von der ersten Kammer (61, 92) unterscheidet, welche für Aufnahme der zu verabrechenden flüssigen Substanz vorgesehen ist und die zu diesem Zweck eine Öffnung (10, 14) enthält, dadurch gekennzeichnet, daß eine dritte Kammer (55) vorgesehen ist, die zwischen den bewegbaren und biegsamen Trennwänden (5, 82) eingegrenzt wird und einen Behälter zum Sammeln der Steuerflüssigkeit und/oder der zu verabrechenden Flüssigkeit für den Fall der Undichtigkeit einer der Trennwände bestimmt und ein Feststellmittel enthält, das eine sichtbare Anzeige im Falle der Undichtigkeit liefert.

2. Anlage nach Anspruch 1, dadurch gekennzeichnet, daß das Feststellmittel auf einer der Trennwände (5, 82) der dritten Kammer befestigt ist.

3. Anlage nach Anspruch 1, dadurch gekennzeichnet, daß das Feststellmittel sich in Freiheit in der dritten Kammer (55) befindet.

4. Anlage nach einem der Ansprüche 1-3, dadurch gekennzeichnet, daß das Feststellmittel in der Lage ist, die Farbe der Flüssigkeit zu modifizieren, die in die dritte Kammer im Falle der Undichtigkeit eindringt.

5. Anlage nach einem der Ansprüche 1-3, dadurch gekennzeichnet, daß das Festgtellmittel in der Lage ist, die Flüssigkeit zu trüben, die im Falle der Undichtigkeit in die dritte Kammer eindringt.

6. Anlage nach Anspruch 5, dadurch gekennzeichnet, daß das Mittel im gasförmigen Zustand ist.

7. Anlage nach einem der Ansprüche 1-6, dadurch gekennzeichnet, daß sie durch eine erste Tasche (61) mit biegsamer Wand (63), die eine zweite dichte Tasche (2) mit doppelter Wand (5, 82) enthält, gebildet wird, und daß die erste Tasche (61) entweder die erste Kammer oder die zweite Kammer bestimmt, während die zweite Tasche (2) die jeweilig andere Kammer bestimmt und daß die doppelte Wand (5, 82) die dritte Kammer (55) bestimmt.

8. Anlage nach Anspruch 7, dadurch gekennzeichnet, daß die Wände der zweiten Kammer durch Vielfach-Trennwände gebildet werden.

9. Anlage nach einem der Ansprüche 1-8, dadurch gekennzeichnet, daß die Öffnungen (10, 14) sich gegenüberliegend angeordnet sind.

10. Anlage nach Anspruch 9, dadurch gekennzeichnet, daß an einem der Enden der Anlage ein Rohrstutzen (14) an der Wand (63) der ersten Tasche (61) abgedichtet verbunden ist und einen Zugang zu der durch diese bestimmten Kammer ermöglicht, während am anderen Ende der Anlage eine Leitung (10) den Zugang zur anderen, von der zweiten Tasche (2) bestimmten Tasche (92) ermöglicht, und daß die doppelte Wand (5, 82) dieser Tasche sowie die Wand (63) der ersten Tasche mit der Leitung (10) abgedichtet verbunden sind.

11. Anlage nach einem der vorhergehenden Ansprüche, dadurch gekennzeichnet, daß mehrere Verbindungsöffnungen in der ersten und der zweiten Kammer vorgesehen und mit dem Äußeren verbunden sind.
